(19) **Europäisches Patentamt**

**European Patent Office**

**Office européen des brevets**

(11) **EP 1 686 136 A1**

(12) **EUROPEAN PATENT APPLICATION**
published in accordance with Art. 158(3) EPC

(43) Date of publication:
02.08.2006 Bulletin 2006/31

(51) Int Cl.:
*C07K 14/75* (2006.01)  *A61K 38/00* (2006.01)
*A61P 35/00* (2006.01)  *A61P 37/04* (2006.01)
*A61P 43/00* (2006.01)  *C12P 21/00* (2006.01)

(21) Application number: 04817163.1

(22) Date of filing: 13.10.2004

(86) International application number:
PCT/JP2004/015057

(87) International publication number:
WO 2005/035571 (21.04.2005 Gazette 2005/16)

(84) Designated Contracting States:
AT BE BG CH CY CZ DE DK EE ES FI FR GB GR
HU IE IT LI LU MC NL PL PT RO SE SI SK TR

(30) Priority: 14.10.2003 JP 2003353419

(83) Declaration under Rule 28(4) EPC (expert solution)

(71) Applicant: Kureha Corporation
Tokyo 103-8552 (JP)

(72) Inventors:
• MATSUNAGA, Kenichi,
c/o Biomedical res lab
Shinjuku-ku, Tokyo 1698503 (JP)
• HOSHI, Hirotaka,
c/o Biomedical res. lab.
Shinjuku-ku, Tokyo 1698503 (JP)

(74) Representative: HOFFMANN EITLE
Patent- und Rechtsanwälte
Arabellastrasse 4
81925 München (DE)

(54) **NOVEL GLYCOPROTEIN AND MEDICINAL COMPOSITION CONTAINING THE SAME**

(57) A novel glycoprotein having (a) a molecular weight of approximately 60 kDa and (b) a ratio of a carbohydrate content to a protein content of 16.4:1.0 (carbohydrate: protein) is disclosed. The glycoprotein can be prepared by (1) extracting *Tricholoma matsutake* with an alkaline solution or hot water, (2) adsorbing the resulting extract by an anion exchange resin, (3) eluting the adsorption fraction from the anion exchange resin with an eluting solution, and (4) obtaining a fraction having a molecular weight of 50 to 70 kDa by gel filtration. The glycoprotein is useful as the active ingredient for an immune enhancing agent, an agent for promoting a recovery from stress, or an antitumor agent.

EP 1 686 136 A1

**Description**

TECHNICAL FIELD

The present invention relates to a novel glycoprotein, and a pharmaceutical composition (for example, a novel immune enhancing agent, a novel agent for promoting a recovery from stress, or a novel antitumor agent) containing the same. The immune enhancing agent, the agent for promoting a recovery from stress, the antitumor agent of the present invention, or the novel glycoprotein of the present invention may be administered as a medicament or in various forms, for example, eatable or drinkable products such as health foods (preferably functional foods) or feeds. The term "foods" as used herein includes drinks. Further, the agent of the present invention may be administered in the form of an oral hygienic composition which is temporarily kept in the mouth but then spat out, retaining almost none of the components, for example, a dentifrice, a mouthwash agent, a chewing gum, or a collutorium, or in the form of an inhalation drawn in through the nose. The term "immuno-enhancing activity" as used herein includes an antitumor activity.

BACKGROUND ART

It is known that *Tricholoma matsutake* (S.Ito & Imai) Sing. contains many physiologically active substances. For example, patent reference 1 and patent reference 2 disclose various antitumor substances contained in *Tricholoma matsutake.* The patent reference 1 discloses that emitanine-5-A, emitanine-5-B, emitanine-5-C, and emitanine-5-D, which are separated and purified from a liquid extract obtained by extracting a broth of *Tricholoma matsutake* mycelia with hot water or a diluted alkaline solution, exhibits an activity of inhibiting a proliferation of sarcoma 180 cells. The patent reference 2 discloses that a protein with a molecular weight of 0.2 to 0.21 million (a molecular weight of a subunit = 0.1 to 0.11 million) which is separated and purified from an extract of *Tricholoma matsutake* fruit bodies with water exhibits an antitumor activity.

Further, the present inventors found that a hot water extract of *Tricholoma matsutake* or an alkaline solution extract of *Tricholoma matsutake,* or an adsorption fraction of a hot water extract of *Tricholoma matsutake* or an alkaline solution extract of *Tricholoma matsutake* by an anion exchange resin exhibits an immuno-enhancing activity (patent reference 3).

Furthermore, the present inventors and coworker found that a partially purified fraction derived from mycelia obtained by culturing the specific *Tricholoma matsutake* strain by the specific culturing method exhibited the immuno-enhancing activity and an activity of promoting a recovery from stress (patent reference 4). In this connection, the partially purified fraction disclosed in the patent reference 4 is different from known fractions, such as the adsorption fraction by an anion exchange disclosed in the patent reference 3, with respect to physicochemical properties.

[patent reference 1] Japanese Examined Patent Publication (Kokoku) No. 57-1230
[patent reference 2] Japanese Patent No. 2767521
[patent reference 3] WO01/49308
[patent reference 4] WO03/070264

DISCLOSURE OF THE INVENTION

PROBLEMS TO BE SOLVED BY THE INVENTION

Although, the present inventors attempted to purify an active substance contained in the above extracts or fractions derived from *Tricholoma matsutake* and having the immuno-enhancing activity and/or the activity of promoting a recovery from stress, it was difficult to purify such an active substance as a single peak, due to the complicated properties of naturally-occurring macromolecular substances (particularly glycoproteins). Under the circumstance, the present inventors conducted intensive studies and, as a result, succeeded in purifying a compound having an immuno-enhancing activity, an activity of promoting a recovery from stress, and an antitumor activity as a single peak, by performing an ion-exchange separation followed by a combination of fractionations on the basis of molecular weight, and further, found that the purified compound was a novel glycoprotein. The present invention is based on the above findings.

Therefore, an object of the present invention is to provide a novel glycoprotein useful as the active ingredient for an immune enhancing agent, an agent for promoting a recovery from stress, and/or an antitumor agent, and to provide a novel pharmaceutical composition containing the glycoprotein, such as an immune enhancing agent, an agent for promoting a recovery from stress, or an antitumor agent.

MEANS FOR SOLVING THE PROBLEMS

The object can be solved by the present invention, i.e., a glycoprotein having the following properties:

(a) molecular weight: approximately 60 kDa (determined by sodium dodecyl sulfate-polyacrylamide gel electrophoresis), and
(b) ratio of carbohydrate content to protein content: 16.4:1.0 (carbohydrate:protein).

Further, the present invention relates to a glycoprotein obtainable by a process comprising the steps of:

(1) extracting *Tricholoma matsutake* with an alkaline solution or hot water,
(2) adsorbing the resulting extract by an anion exchange resin,
(3) eluting the adsorption fraction from the anion exchange resin with an eluting solution, and
(4) fractionating from the eluted fraction obtained by the eluting step a fraction having a molecular weight of 50 to 70 kDa by gel filtration.

The present invention relates to a pharmaceutical composition comprising the above glycoprotein, and a pharmaceutically or veterinarily acceptable carrier or diluent.
The present invention relates to an immune enhancing agent, an agent for promoting a recovery from stress, or an antitumor agent, characterized by comprising as an active ingredient the above glycoprotein.
The present invention relates to a method for an immune enhancement, comprising administering to a subject in need thereof the above glycoprotein in an amount effective therefor.
The present invention relates to a method for promoting a recovery from stress, comprising administering to a subject in need thereof the above glycoprotein in an amount effective therefor.
The present invention relates to a method for treating or preventing a tumor, comprising administering to a subject in need thereof the above glycoprotein in an amount effective therefor.
The present invention relates to use of the above glycoprotein in the manufacture of an immune enhancing agent or a pharmaceutical composition for an immune enhancement, an agent for promoting a recovery from stress or a pharmaceutical composition for promoting a recovery from stress, or an antitumor agent or a pharmaceutical composition for treating or preventing a tumor.

EFFECTS OF THE INVENTION

According to the immune enhancing agent of the present invention, an immune activity can be enhanced in a subject in need of an immune enhancement. Further, according to the agent of the present invention for promoting a recovery from stress, a recovery from a loading of stress can be promoted. Furthermore, according to the antitumor agent of the present invention, a tumor can be treated or prevented. The glycoprotein of the present invention is useful as the active ingredient for the immune enhancing agent, the agent for promoting a recovery from stress, or the antitumor agent.

BEST MODE FOR CARRYING OUT THE INVENTION

[1] Glycoprotein of the present invention
The glycoprotein of the present invention exhibits the following properties.

(1) Molecular weight: The molecular weight determined by sodium dodecyl sulfate-polyacrylamide gel electrophoresis (SDS-PAGE) is approximately 60 kDa (50 to 70 kDa).
(2) Protein content and carbohydrate content: The content of proteins is 38.42 $\mu$g/mg, on the basis of amounts of amino acids resulting from a hydrolysis of the glycoprotein of the present invention. The content of carbohydrates is 628.72 $\mu$g/mg, on the basis of amounts of monosaccharides resulting from the hydrolysis of the glycoprotein of the present invention. The ratio of the carbohydrate content to the protein content is 16.4:1.0 (carbohydrate:protein).
(3) Fatty acid content: Stearic acid (C18:0) 1.86 $\mu$g/mg, palmitic acid (C16:0) 1.84 $\mu$g/mg, oleic acid [C18:1(9)] 1.52 $\mu$g/mg, linoleic acid [C18:2(9,12)] 0.94 $\mu$g/mg, and myristic acid (C14:0) 0.55 $\mu$g/mg.
(4) Amino acid composition: Aspartic acid and asparagine 9.65 mol%, threonine 6.15 mol%, serine 6.83 mol%, glutamic acid and glutamine 10.19 mol%, glycine 8.74 mol%, alanine 9.84 mol%, valine 6.94 mol%, 1/2-cystine 0.15 mol%, methionine 1.31 mol%, isoleucine 5.47 mol%, leucine 9.55 mol%, tyrosine 2.64 mol%, phenylalanine 4.09 mol%, lysine 4.99 mol%, histidine 1.97 mol%, arginine 5.00 mol%, tryptophan 1.17 mol%, and proline 5.33 mol%.

(5) Neutral sugar composition: Glucose 561.45 $\mu$g/mg, mannose 16.68 $\mu$g/mg, galactose 42.65 $\mu$g/mg, and fucose 9.57 $\mu$g/mg. Xylose is less than a detectable limit.

(6) Amino sugar composition: Glucosamine 0.36 $\mu$g/mg. Mannosamine is less than a detectable limit.

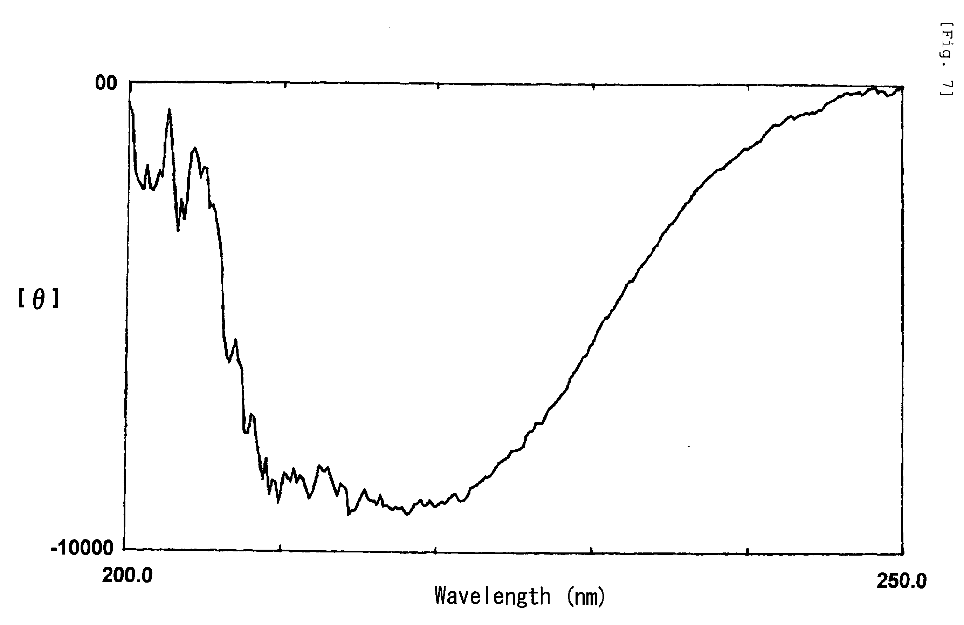

(7) Circular dichroism analysis (CD): The spectrum is shown in Figure 7.

(8) Nuclear magnetic resonance (NMR) analysis

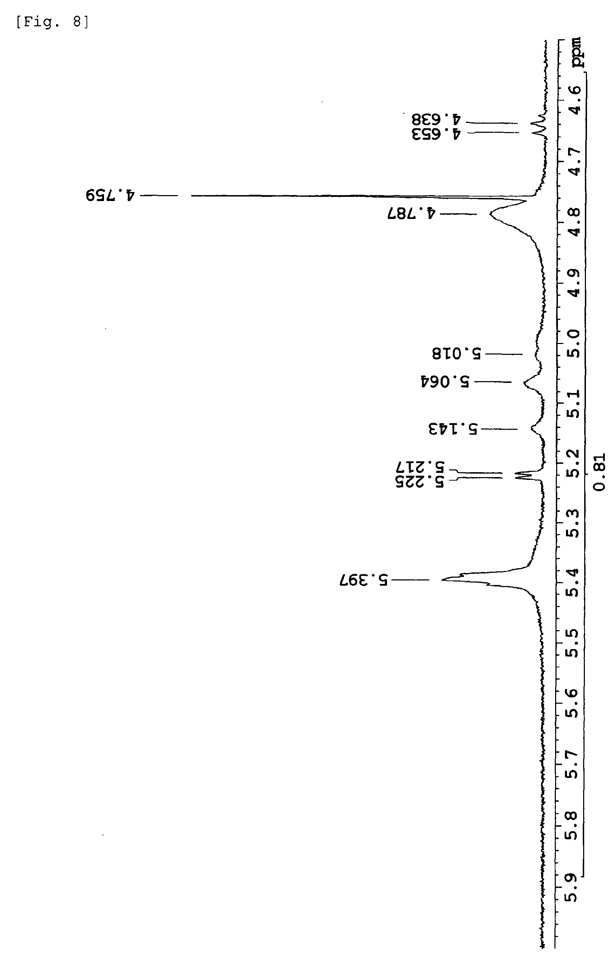

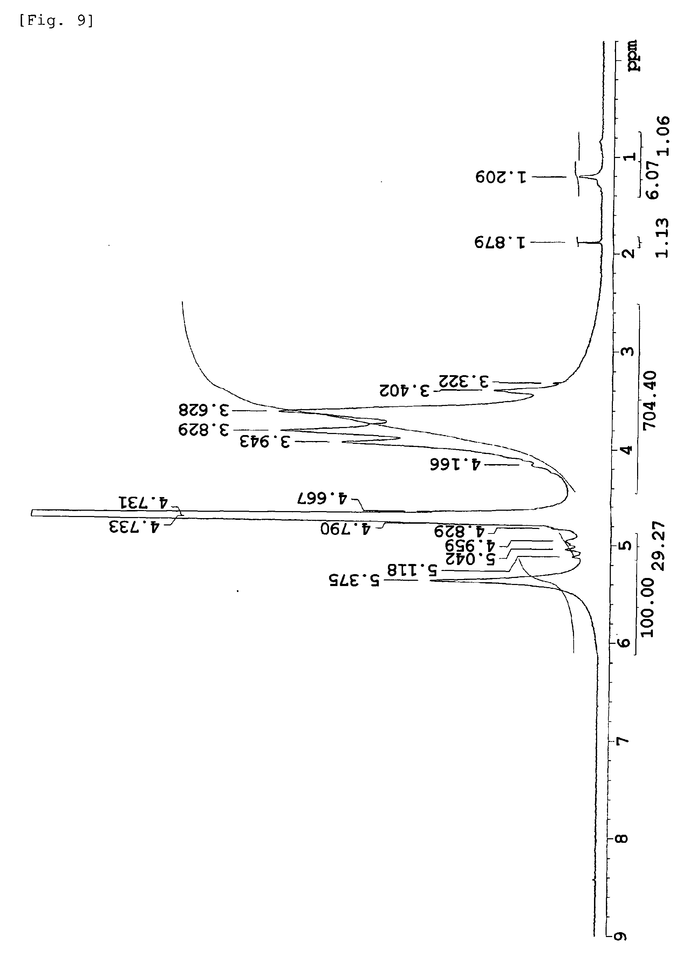

    (i) $^1$H one-dimensional NMR analysis: The spectrum of the glycoprotein of the present invention is shown in Figure 8. The spectrum when the glycoprotein is treated with hydrazine to remove the protein portion is shown in Figure 9.

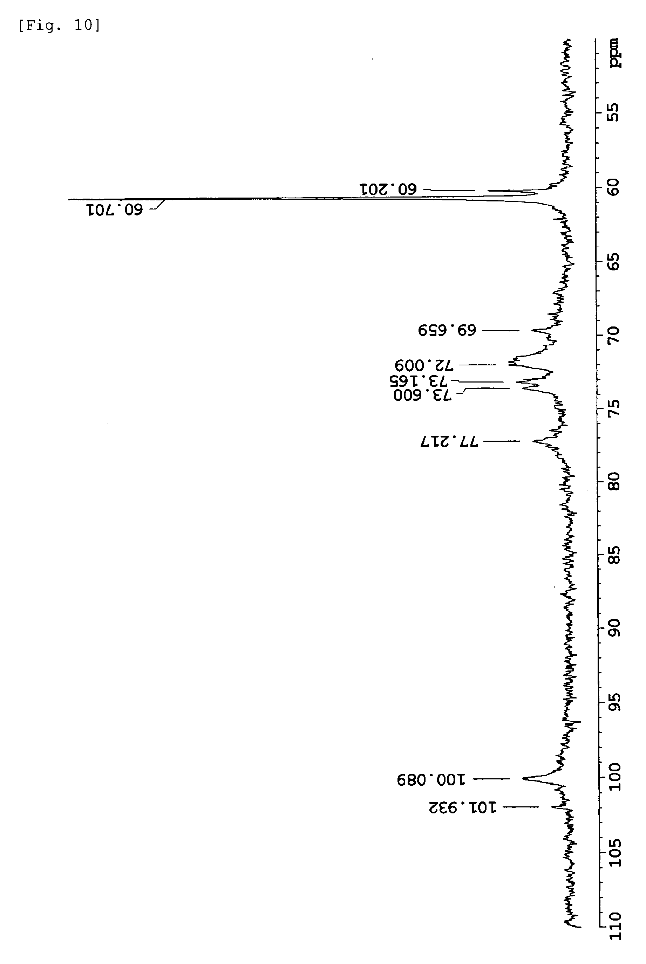

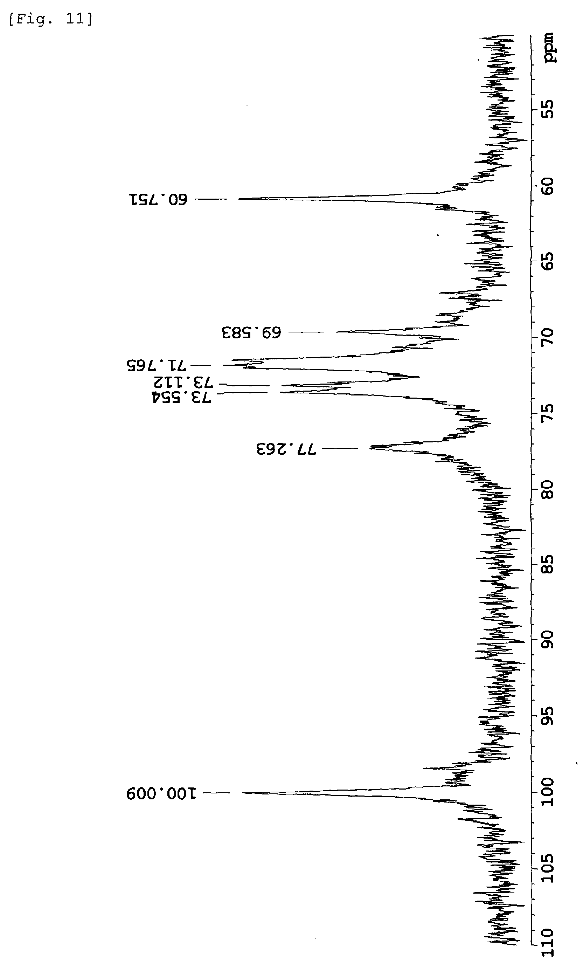

    (ii) $^{13}$C one-dimensional NMR analysis: The spectrum of the glycoprotein of the present invention is shown in Figure 10. The spectrum when the glycoprotein is treated with hydrazine to remove the protein portion is shown in Figure 11.

(9) Carbohydrate binding position in carbohydrate portion: The carbohydrate portion in the glycoprotein of the present invention contains each binding type as shown in Table 6, at the ratio as shown in Table 6. The major binding in the glycoprotein is 1→4 linked glucose.

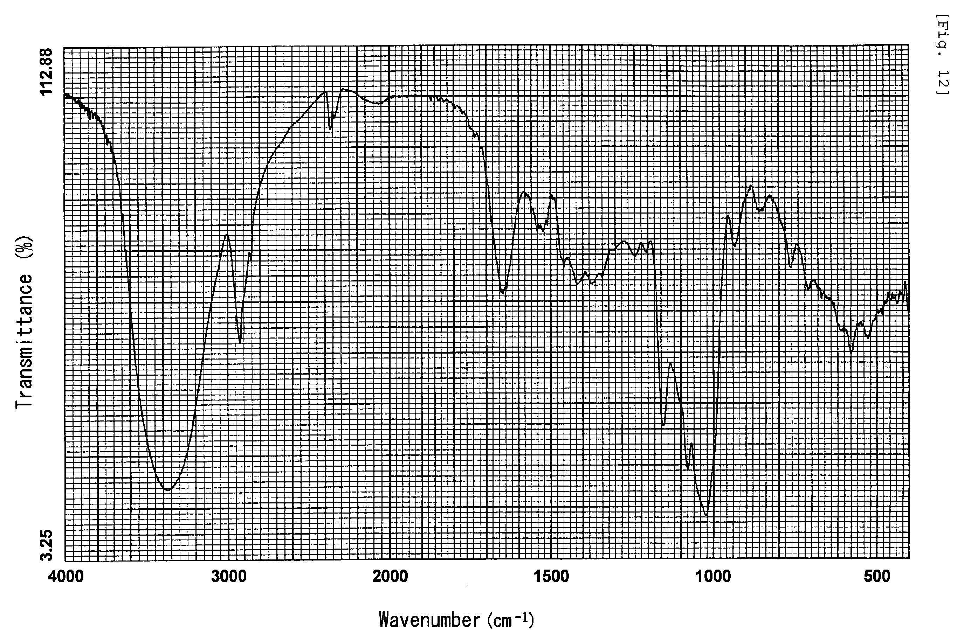

(10) Infrared spectroscopic analysis (IR): The spectrum is shown in Figure 12.

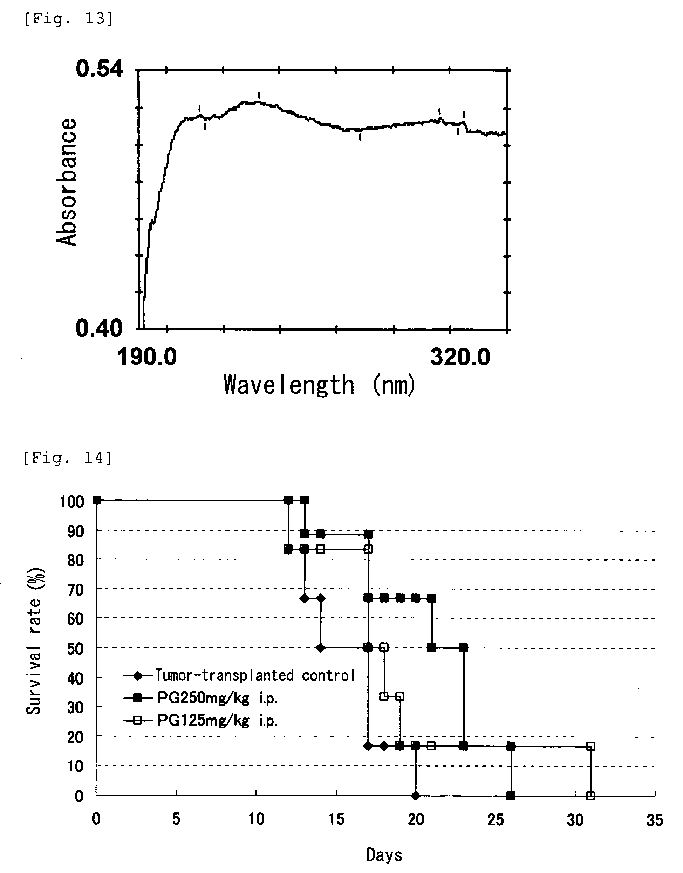

(11) Ultraviolet spectroscopic analysis (UV): The spectrum is shown in Figure 13.

The origin of the glycoprotein of the present invention is not particularly limited, so long as the glycoprotein exhibits the above properties. The glycoprotein of the present invention can be prepared from, for example, *Tricholoma matsutake.*

As the *Tricholoma matsutake,* there may be mentioned, for example, a fruit body or a mycelium of a naturally occurring *Tricholoma matsutake,* or a mycelium or a broth obtainable by culturing *Tricholoma matsutake.* The *Tricholoma matsutake* used for the cultivation may be, for example, the *Tricholoma matsutake* strain FERM BP-7304 disclosed in WO02/30440. The *Tricholoma matsutake* strain FERM BP-7304 was deposited in the International Patent Organism Depositary National Institute of Advanced Industrial Science and Technology [(Former Name) National Institute of Bioscience and Human-Technology Agency of Industrial Science and Technology (Address: AIST Tsukuba Central 6, 1-1, Higashi 1-chome Tukuba-shi, Ibaraki-ken 305-8566 Japan)] on September 14, 2000. The *Tricholoma matsutake* strain FERM BP-7304 was established as a strain by culturing a piece of fruit body from a *Tricholoma matsutake* strain CM6271 collected in Kameoka-shi, Kyoto, Japan and then culturing it in vitro, and is maintained in the Biomedical Research Laboratories, Kureha Chemical Industry Co. Ltd.

The glycoprotein of the present invention may be prepared by, for example, but is by no means limited to, a process comprising the steps of:

extracting *Tricholoma matsutake* with an alkaline solution or hot water (hereinafter referred to as the extracting step); adsorbing the resulting extract by an anion exchange resin (hereinafter referred to as the anion exchange resin-adsorbing step);

eluting the adsorption fraction from the anion exchange resin with an appropriate eluting solution (hereinafter referred to as the eluting step); and

fractionating, from the fraction eluted under the eluting conditions as described below in the above eluting step, a fraction having a molecular weight of 50 to 70 kDa by gel filtration (hereinafter referred to as the gel filtration fractionating step). In this connection, the glycoprotein of the present invention includes not only the glycoprotein prepared by the above process, but also a glycoprotein prepared by a process other than the above process, so long as it exhibits the same properties.

In the extracting step, when fruit bodies or mycelia of *Tricholoma matsutake* are used, it is preferable to crush, grind, or pulverize the fruit bodies or mycelia, to enhance the extraction efficiency.

When hot water is used in the extracting step, the temperature of hot water is preferably 60 to 100°C, more preferably 80 to 98°C. It is preferable to carry out the extraction with stirring or shaking, so that the extraction efficiency is enhanced.

An extracting time may be appropriately determined in accordance with, for example, the form of *Tricholoma matsutake* (for example, a fruit body, mycelia, or a broth; or a crushed, ground, or pulverized form, if treated), the temperature of the hot water, or with or without stirring or shaking or a treating condition thereof, but is generally 1 to 6 hours, preferably 2 to 3 hours.

When an alkaline solution is used in the extracting step, the alkaline solution may be, for example, but is by no means limited to, an aqueous solution of a hydroxide of an alkaline metal (such as sodium or potassium), particularly sodium hydroxide. The alkaline solution is preferably pH 8 to 13, more preferably pH 9 to 12. The alkaline solution extraction may be carried out preferably at 0 to 20°C, more preferably at 0 to 15°C. The resulting alkaline solution extract may be neutralized before the next anion exchange resin-adsorbing step is carried out.

The extract obtained in the extracting step may be used, without a further treatment (i.e., together with insolubles), in the next anion exchange resin-adsorbing step. However, it is preferable to remove the insolubles from the extract, or to remove the insolubles followed by a low molecular fraction therefrom, before the next anion exchange resin-adsorbing step is carried out. For example, the extract in which insolubles are contained may be centrifuged to remove the insolubles, and the resulting supernatant may be used in the next anion exchange resin-adsorbing step. Alternatively, the supernatant obtained by centrifuging the extract containing insolubles may be dialyzed to remove a low molecular fraction (preferable a fraction of substances having a molecular weight of 3500 or less), and the resulting dialyzate may be used in the next anion exchange resin-adsorbing step.

The extract obtained in the extracting step may be applied to a defatting step, before the next anion exchange resin-adsorbing step. In this connection, the defatting step or the above insoluble-removing step and/or low-molecular-fraction-removing step can be carried out alone, or as a combination thereof.

As an organic solvent used in the defatting step, there may be mentioned, for example, fat-soluble organic solvents (for example, chloroform, methanol, ether, ethanol, methyl acetate, or hexane) or a combination thereof (for example, a mixture of chloroform and methanol). A mixture of chloroform and methanol is preferable. When a mixture of chloroform and methanol is used as the organic solvent, the mixing ratio [chloroform:methanol (v/v)] may be, for example, 10:1 to 1:10. It is preferable to carry out the defatting step at 15 to 30°C.

As an anion exchange resin which may be used in the anion exchange resin-adsorbing step, a known anion exchange resin, for example, diethylaminoethyl (DEAE) cellulose or triethylaminoethyl (TEAE) cellulose, may be used.

An eluting solution used in the eluting step may be appropriately selected in accordance with an anion exchange resin used in the anion exchange resin-adsorbing step. For example, an aqueous solution of sodium chloride may be used. Among the fractions eluted in the eluting step, the fraction eluted under the eluting conditions as described below (for example, the Fraction B in Example 2 described below) may be used in the next gel filtration fractionating step.

When the adsorbing step is carried out, for example, under the conditions in which a DEAE TOYOPEARL PAK 650M packed column ($\varphi$ 22 mm, h 20 cm) is used as the anion exchange resin, and a sample to be adsorbed is applied as a solution dissolved in 50 mmol/L Tris-HCl buffer (pH7.2), the eluting step may be carried out as follows. That is, a linear gradient using an eluting liquid A (50 mmol/L Tris-HCl buffer) and an eluting liquid B (50 mmol/L Tris-HCl buffer containing 1 mol/L NaCl) may be carried out at a flow rate of 5 mL/min, and a fraction eluted in the range of 12 to 15 minutes (liquid volume = 15 mL) may be used in the next gel filtration fractionating step.

The gel filtration fractionating step may be carried out by a conventional method. A fraction containing the glycoprotein of the present invention as a single peak can be obtained by collecting a fraction having a molecular weight of 50 to 70 kDa (such as the fraction B-1-2 in Example 2 described below). The gel filtration fractionating step can be carried out using plural gel filtration columns (such as 2 types of gel filtration columns) having different fraction ranges, at plural steps (such as two steps). For example, a gel filtration column having a fraction range of $1\times10^3$ to $1\times10^5$ Da (MW) is used to collect a fraction having a molecular weight of 10 kDa or more (such as fraction B-1 in Example 2), and the collected fraction is applied to a gel filtration column having a fraction range of $4\times10^4$ to $2\times10^7$ Da (MW) to obtain a fraction having a molecular weight of 50 to 70 kDa (such as the fraction B-1-2 in Example 2).

[2] Pharmaceutical composition of the present invention

The pharmaceutical composition of the present invention (particularly, the immune enhancing agent, the agent for promoting a recovery from stress, or the antitumor agent of the present invention) contains the glycoprotein of the present invention as the active ingredient.

In the immune enhancing agent, the agent for promoting a recovery from stress, or the antitumor agent of the present invention, the glycoprotein of the present invention, as the active ingredient, may be administered to an animal (preferably a mammal, particularly a human) with a pharmaceutically or veterinarily acceptable ordinary carrier or diluent. That is, the composition (preferably pharmaceutical composition) of the present invention [for example, the composition for an immune enhancement (preferably the pharmaceutical composition for an immune enhancement), the composition for promoting a recovery from stress (preferably the pharmaceutical composition for promoting a recovery from stress), or the composition for treating or preventing a tumor (preferably the pharmaceutical composition for treating or preventing a tumor)] contains the glycoprotein of the present invention, as the active ingredient, and a pharmaceutically or veterinarily acceptable carrier or diluent.

The active ingredient in the immune enhancing agent of the present invention, i.e., the glycoprotein of the present invention, exhibits an immuno-enhancing activity. The immuno-enhancing activity includes, for example, various activities disclosed in WO03/070264, and an activity to inhibit a TGF-β (an immunosuppressive substance) activity. WO03/070264 discloses an antitumor activity [for example, an activity to extend a survival time in a subject with cancer, an anti-primary tumor activity (particularly an activity to inhibit a primary tumor proliferation), or an antimetastasis activity (particularly an activity to inhibit a metastatic focus proliferation)], an activity to produce an induction of a killer activity (particularly an activity to produce an induction of a killer activity of an intestinal lymphocyte), an activity to enhance recognition of a tumor cell, an activity to enhance a gene expression of interleukin 12 (IL-12), and an activity to increase a serum IAP value.

As the diseases to be treated or prevented with the immune enhancing agent of the present invention, there may be mentioned, for example, cancer, infectious diseases, autoimmune diseases, chronic fatigue syndrome, or life-style related diseases.

The active ingredient in the present invention, the glycoprotein of the present invention, may be administered alone or, preferably, together with a pharmaceutically or veterinarily acceptable ordinary carrier or diluent to a subject in need of an immune enhancement in an amount effective therefor.

Further, the active ingredient in the present invention, the glycoprotein of the present invention, may be used in the manufacture of an immune enhancing composition (preferably an immune enhancing pharmaceutical composition), an immune enhancing health food (preferably an immune enhancing functional food), or an oral hygienic composition for an immune enhancement.

The active ingredient in the agent of the present invention for promoting a recovery from stress, i.e., the glycoprotein of the present invention, exhibits an activity of promoting a recovery from stress. As the diseases to be treated or prevented with the agent of the present invention for promoting a recovery from stress, there may be mentioned, for example, cancer, infectious diseases, autoimmune diseases, chronic fatigue syndrome, or life-style related diseases. The active ingredient in the present invention, the glycoprotein of the present invention, may be administered alone or, preferably, together with a pharmaceutically or veterinarily acceptable ordinary carrier or diluent to a subject in need of promoting a recovery from stress in an amount effective therefor.

Further, the active ingredient in the present invention, the glycoprotein of the present invention, may be used in the manufacture of a composition for promoting a recovery from stress (preferably a pharmaceutical composition for promoting a recovery from stress), a health food for promoting a recovery from stress (preferably a functional food for promoting a recovery from stress), or an oral hygienic composition for promoting a recovery from stress.

Generally, when stress is loaded to an animal once or during a certain period, an immune activity in the animal is decreased but the immune activity is spontaneously recovered after a release of the stress. The "activity of promoting a recovery from stress" as used herein means an activity which promotes the recovery of an immune activity during a period of an immune activity convalescence after a release of stress, in comparison with the spontaneous recovery.

The agent of the present invention for promoting a recovery from stress can be administered at any time, so long as it can promote the recovery of an immune activity briefly lowered by stress. It can be administered, for example, before a loading of stress, during a loading of stress, and/or during a period of an immune activity convalescence after a release of stress.

In this connection, the "activity of promoting a recovery from stress" in the present invention is different from the above-described mere "immuno-enhancing activity" found by the present inventor. Generally, the "immuno-enhancing activity" means an activity in which an enhancement of an immune activity is observed by administering an active ingredient having such an activity in comparison with a state before the administration, that is, an activity of enhancing an immune activity per se. The state before the administration may be a state in which an immune activity is natural or lowered by stress. In contrast, the "activity of promoting a recovery from stress" in the present invention is an activity which promotes the recovery of an immune activity during a period of an immune activity convalescence, as described above, that is, an activity of enhancing the speed of recovery of an immune activity. When the agent of the present invention for promoting a recovery from stress is administered, the speed of recovery of an immune activity is increased in comparison with the case in which the agent of the present invention for promoting a recovery from stress is not administered.

Further, in the "immuno-enhancing activity", enhancement of an immune activity is directly observed when administering an active ingredient having such an activity. In contrast, when administering the active ingredient of the agent of the present invention for promoting a recovery from stress (i.e., the glycoprotein of the present invention) to a subject animal before a loading of stress, the recovery of an immune activity is promoted during a period of an immune activity convalescence, even if the glycoprotein of the present invention is not administered during a loading of stress and during a period of an immune activity convalescence. With respect to the point, the "activity of promoting a recovery from stress" in the present invention is different from the "immuno-enhancing activity" in the present invention.

The active ingredient in the antitumor agent of the present invention, i.e., the glycoprotein of the present invention, exhibits an antitumor activity. The term "antitumor activity" as used herein is included in the immuno-enhancing activity. The antitumor activity includes, for example, an activity to extend a survival time in a subject with cancer, an anti-primary tumor activity (particularly an activity to inhibit a primary tumor proliferation), or an antimetastasis activity (particularly an activity to inhibit a metastatic focus proliferation). As cancer to be treated or prevented with the antitumor agent of the present invention, there may be mentioned, for example, lung cancer, stomach cancer, liver cancer, colorectal cancer, pancreatic cancer, esophagus cancer, breast cancer, uterine cancer, prostatic cancer, sarcoma, melanoma, or leukemia.

The active ingredient in the present invention, the glycoprotein of the present invention, may be administered alone or, preferably, together with a pharmaceutically or veterinarily acceptable ordinary carrier or diluent to a subject in need of treating or preventing a tumor in an amount effective therefor.
Further, the active ingredient in the present invention, the glycoprotein of the present invention, may be used in the manufacture of an antitumor composition (preferably an antitumor pharmaceutical composition), an antitumor health food (preferably an antitumor functional food), or an oral hygienic composition for treating or preventing a tumor.

The formulation of the pharmaceutical composition of the present invention is not particularly limited to, but may be, for example, oral medicines, such as powders, fine particles, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants, such as sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate.

The parenteral administration may be, for example, an injection such as a subcutaneous or intravenous injection, or a per rectum administration. Of the parenteral formulations, an injection is preferably used.
When the injections are prepared, for example, watersoluble solvents, such as physiological saline or Ringer's solution, water-insoluble solvents, such as plant oil or fatty acid ester, agents for rendering isotonic, such as glucose or sodium chloride, solubilizing agents, stabilizing agents, antiseptics, suspending agents, or emulsifying agents may be optionally used, in addition to the active ingredient.
The pharmaceutical composition of the present invention may be administered in the form of a sustained release preparation using sustained release polymers. For example, the pharmaceutical composition of the present invention may be incorporated to a pellet made of ethylenevinyl acetate polymers, and the pellet may be surgically implanted in a tissue to be treated.

The pharmaceutical composition of the present invention may contain the glycoprotein of the present invention in an amount of, but is by no means limited to, 0.01 to 99% by weight, preferably 0.1 to 80% by weight.
A dose of the pharmaceutical composition of the present invention is not particularly limited, but may be determined dependent upon the kind of disease, the age, sex, body weight, or symptoms of the subject, a method of administration, or the like. The pharmaceutical composition of the present invention may be orally or parenterally administered.

The agent of the present invention may be administered as a medicament or in various forms, for example, eatable or drinkable products such as health foods (preferably functional foods) or feeds. The term "foods" as used herein includes drinks.
It is known that food has (1) a function as nutrients (the first function), (2) a function which appeals to five senses in a human (the second function), and (3) a function which has a favorable influence on the health, physical ability, or mental condition in a human (the third function). The third function regulates various physiological systems, such as the digestive system, circulatory system, endocrine system, immune system, or nervous system, and has a favorable influence on the maintaining or recovery of health. The term "health food" as used herein means food which provides, or is expected to provide, one or more effects on health. The term "functional food" as used herein means processed or designed food such that the above various biological regulatory functions (i.e., functions which regulate physiological systems such as the digestive system, circulatory system, endocrine system, immune system, or nervous system) may be fully expressed.

Further, the agent of the present invention may be administered in the form of an oral hygienic composition which is temporarily kept in the mouth but then spat out, retaining almost none of the components, for example, a dentifrice, a mouthwash agent, a chewing gum, or a collutorium, or in the form of an inhalation drawn in through the nose. More particularly, the glycoprotein of the present invention may be added to a desired food including a drink, a feed, a dentifrice, a mouthwash agent, a chewing gum, a collutorium, or the like as an additive, such as a food additive.

EXAMPLES

The present invention now will be further illustrated by, but is by no means limited to, the following Examples.

Example 1: Methods for evaluating activities

In Example 2 mentioned below, the following methods for evaluating (1) an activity for promoting a recovery of a natural killer (NK) cell activity from stress and (2) a binding activity to human recombinant transforming growth factor-$\beta_1$ (TGF-$\beta_1$) were used for the evaluation of mycelia of *Tricholoma matsutake* FERM BP-7304 and fractions derived from the mycelia.

(1) Evaluation for activity for promoting recovery of NK cell activity from stress

The activity for promoting a recovery of an NK cell activity from stress was evaluated by the following procedure. That is, after each sample for evaluation was orally administered to mice for 10 days, restraint stress was loaded for 18 hours, and then the NK cell activity was measured after a release of the stress to examine the effects of the sample.

More particularly, an aqueous solution of each sample for evaluation was orally administered to 8-week-old male C57BL/6 mice (purchased from Charles River Japan; 5 mice per group) at a dose of 300 mg/kg/day (as mycelia of *Tricholoma matsutake)* for 10 days in normal breeding cages. After the administration for 10 days, mice were transferred from the normal breeding cages to 50 mL-capped polypropylene centrifuge tubes (catalog No. 2341-050; Asahi Techno Glass Corporation) with air vents so that a mouse was confined in a tube. The confined mice could not move in the tubes. The tubes in which mice were confined were placed in the cages and allowed to stand for 18 hours to load the mice with restraint stress. After the stress loading for 18 hours, mice were transferred from the tubes to the breeding cages, and bred under ordinary breeding conditions.

After 7 days had passed from the release of the restraint stress, mice were sacrificed, and a natural killer (NK) cell activity was evaluated by measuring a cytotoxic activity of lymphocytes against an NK-sensitive tumor cell line YAC-1 in vitro, in accordance with a method of Greenberg et al. (Greenberg A.H. et al., J.Exp.Psychol., 12, 25-31, 1986) as follows.

Spleens were aseptically taken from mice and transferred to a sterile petri dish containing a Hanks balanced salt solution. The lymph nodes were teased with scissors and tweezers, and passed through a mesh to prepare a suspension containing single lymphocyte cells. The cells were washed three times with an RPMI 1640 medium containing a 10% bovine fetal serum which had been heated at 56°C for 30 minutes. Then, a concentration of cells was adjusted to $5 \times 10^6$/mL with an RPMI 1640 medium containing 20 mmol/L of 4-(2-hydroxyethyl)-1-piperazine ethanesulfonate and 20 $\mu$g/mL of gentamicin. The resulting cell suspension was used as an effector cell.

The YAC-1 cell used as a target cell was maintained in an RPMI 1640 medium containing a 10% bovine fetal serum which had been heated at 56°C for 30 minutes, at Biomedical Research Laboratories, Kureha Chemical Industry Co. Ltd. The YAC-1 cells were reacted with radioactive sodium chromate (Amersham Japan) at 37°C for 20 minutes. Unreacted radioactive sodium chromate was removed by washing three times with an RPMI 1640 medium containing a 10% bovine fetal serum which had been heated at 56°C for 30 minutes, and a concentration of tumor cells labeled with radioactive chromium was adjusted to $5 \times 10^4$/mL.

0.1 mL of the effector cell suspension or a double-diluted series thereof and 0.1 mL of the suspension of tumor cells labeled with radioactive chromium were put into a test tube, and reacted in a 5% carbon dioxide gas incubator at 37°C for 4 hours. In this connection, to calculate a "specific lysis" described below, a suspension prepared by putting the tumor cells labeled with radioactive chromium and a medium into a test tube, and a suspension prepared by putting the tumor cells labeled with radioactive chromium and a detergent (Triton; a final concentration = 0.05%) into a test tube were also reacted in a 5% carbon dioxide gas incubator at 37°C for 4 hours. After the reaction was completed, 1.5 mL of an RPMI 1640 medium containing a 10% bovine fetal serum, which had been heated at 56°C for 30 minutes, was added to each test tube, and thoroughly mixed by a mixer. The whole was centrifuged at 1200 rpm for 5 minutes at 4°C to obtain a supernatant, and the radioactivity was measured by a gamma counter.

A specific lysis (S.L.; unit = %) was calculated by an equation:

$$[S.L.] = \{ (B - B_f) / (B_{max} - B_f) \} \times 100$$

wherein B is a radioactivity (unit = Bq) of a supernatant of an experimental group, $B_f$ is a radioactivity (unit = Bq) of a supernatant of a spontaneously releasing group, and $B_{max}$ is a radioactivity (unit = Bq) of a supernatant of a maximum releasing group. The NK cell activity was represented by "Lytic Units 30% (LU30)", that is, a number of cells which kill 30% tumor cells per $10^7$ cells of effector cells. When the NK cell activity of the sample for evaluation was significantly promoted in comparison with that in the sterile water-administered control group ($p < 0.05$), the result is shown as "Active".

When no significant difference was observed (p≥0.05), the result is shown as "Not active".

(2) Evaluation for TGF-$\beta_1$ binding activity

The TGF-$\beta_1$ binding activity was evaluated by reacting each sample for evaluation with a TGF-$\beta_1$ preparation in a test tube for 2 hours and measuring the complex of each sample and TGF-$\beta_1$ [the binding activity of each sample with TGF-$\beta$1] using an enzyme immunoassay.

In a polypropylene tube (multi-siliconized tube, Safe Seal Microcentrifuge Tube; Funakoshi) which slightly adsorbed proteins, a TGF-$\beta_1$ preparation (Funakoshi) was dissolved in a phosphate buffer (pH 7.2) containing 2% albumin, and the solution was adjusted to 100 ng/mL. Further, each sample for evaluation was dissolved in a phosphate buffer containing 2% albumin, and the solution was adjusted to 2 mg/mL. Into a tube which slightly adsorbed proteins, 0.5 mL of the TGF-$\beta_1$ solution and 0.5 mL of a diluted series of the sample solution were charged, and the reaction was performed at 22°C for 3 hours. After the reaction was completed, the content of TGF-$\beta_1$ in the reaction solution was measured using a commercially available measuring kit (Quantikine Human TGF-$\beta_1$ ELISA Kit; Funakoshi).

The binding rate (unit = %) was calculated by the following equation:

$$\texttt{[Binding ratio (\%)] = \{(Tc-T)/Tc\} \times 100}$$

wherein T is the found value (unit = ng/mL) of TGF-$\beta_1$ in the group wherein the sample for evaluation was administered, and Tc is the found value (unit = ng/mL) of TGF-$\beta_1$ in the group wherein the phosphate buffer containing 2% albumin was administered. When the TGF-$\beta_1$ binding rate of the sample for evaluation was 30% or more, the result is shown as "Active". When the TGF-$\beta_1$ binding rate of the sample for evaluation was 30% or less, the result is shown as "Not active".

Example 2: Isolation of active fractions from dry powder of *Tricholoma matsutake* FERM BP-7304 mycelia

To a 500-mL flask, 2.0 g of dry powder (lot No. M65T1Y1) of *Tricholoma matsutake* FERM BP-7304 mycelia was added and extracted with 100 mL of 0.2 mol/L sodium hydrate at 23°C for 1 hour while stirring. The whole was centrifuged at 12000 rpm for 30 minutes at 4°C to collect the supernatant (i.e., alkaline solution extract) and the pellets (i.e., alkaline extraction residue). In this connection, the pH was adjusted to 7.0 with 1.0 mol/L HCl. The resulting alkaline solution extract was transferred to a 500-mL separating funnel, and extracted with 100mL of a mixture of chloroform and methanol [1:1(v/v); hereinafter referred to as ChMe] at 23°C. The aqueous layer and the ChMe layer were independently collected and lyophilized, to obtain 0.5 g and 0.05 g of residues as powder, respectively. The biological activities were detected in the aqueous layer (Table 1).

Table 1

| No. | Sample | TGF-$\beta_1$ binding activity | Activity for promoting NK cell recovery |
|---|---|---|---|
| 1 | a | (N.D.) | At 300 mg/kg; Active |
| 2 | b | At 100 mg/mL; Active | At 150 mg/kg; Active |
| 3 | c | (N.D.) | At 300 mg/kg; Not active |
| 4 | d | At 100 mg/mL; Active | At 150 mg/kg; Active |
| 5 | e | At 100 mg/mL; Not active | At 300 mg/kg; Not active |
| 6 | f | At 100 mg/mL; Active | At 150 mg/kg; Active |

a: BP-7304 dry powder (starting substance)

b: Alkaline solution extract

c: Alkaline extraction residue

d: Aqueous layer obtained by treating the alkaline solution extract with ChMe

e: ChMe layer obtained by treating the alkaline solution extract with ChMe

f: Reassembly (sample No. 4 and sample No. 5)

N.D.: Not determined

The powder (0.5 g) of the aqueous layer was dissolved in 5 mL of 0.05 mol/L Tris-HCl buffer (pH7.2). The solution was applied to a diethylaminoethyl (DEAE) TOYOPEARL PAK 650M packed column (φ 22 mm, h 20 cm) which had been equilibrated with the same buffer, and eluted by a linear gradient using an eluting liquid A (50 mmol/L Tris-HCl buffer) and an eluting liquid B (50 mmol/L Tris-HCl buffer containing 1 mol/L NaCl) at a flow rate of 5 mL/min. The fractionation was carried out using an absorbance at 280 nm as an index.

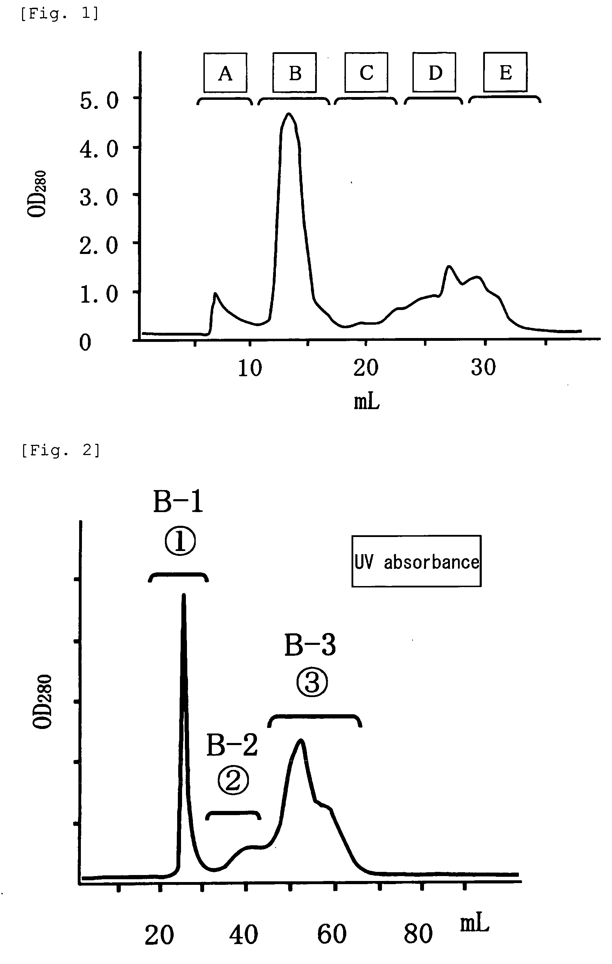

The resulting elution pattern is shown in Figure 1. Peaks were divided into five fractions. Each fraction was dialyzed in distilled water for more than 20 hours using a dialysis membrane having a molecular weight cutoff of 13000. The biological activities of each dialyzate were measured, and as a result, the activities were detected in the fraction B (Table 2).

Table 2

| No. | Sample | TGF-$\beta_1$ binding activity | Activity for promoting NK cell recovery |
|---|---|---|---|
| 1 | a | (N.D.) | At 300 mg/kg; Active |
| 2 | b | At 100 mg/mL; Not active | At 300 mg/kg; Not active |
| 3 | c | At 30 mg/mL; Active | At 65 mg/kg; Active |
| 4 | d | At 100 mg/mL; Active | At 300 mg/kg; Not active |
| 5 | e | At 100 mg/mL; Not active | At 300 mg/kg; Not active |
| 6 | f | At 100 mg/mL; Not active | At 300 mg/kg; Not active |

a: BP-7304 dry powder (positive control)

b: Fraction A obtained by the DEAE chromatography

c: Fraction B obtained by the DEAE chromatography

d: Fraction C obtained by the DEAE chromatography

e: Fraction D obtained by the DEAE chromatography

f: Fraction E obtained by the DEAE chromatography

N.D.: Not determined

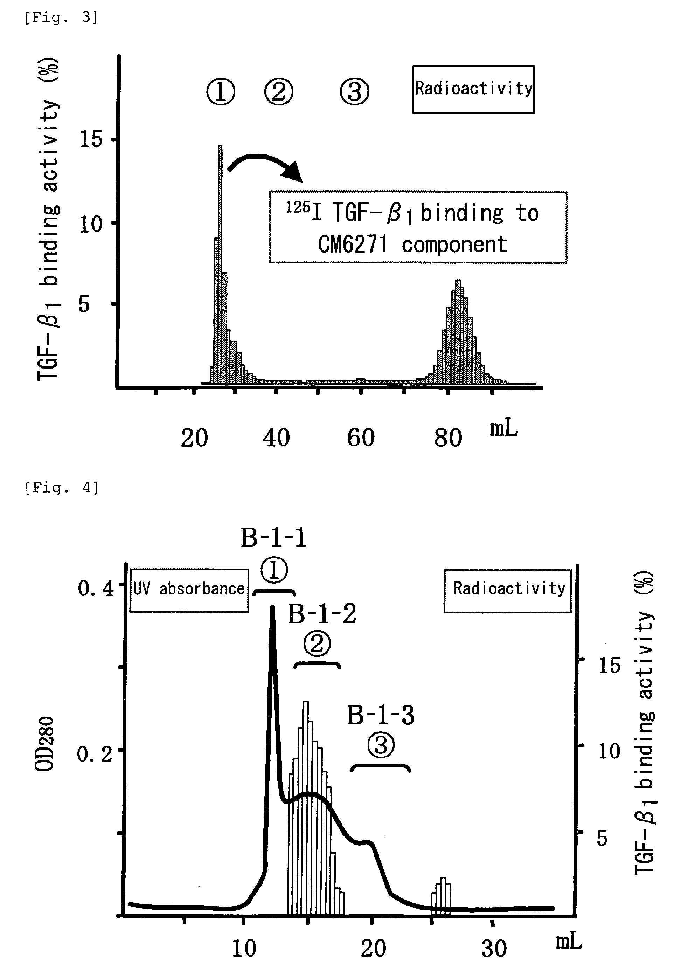

The dialyzate derived from the faction B was applied to a Sephacryl S-100 (Pharmacia) packed column ($\varphi$ 16 mm, h 300 mm) which had been equilibrated with 0.05 mol/L Tris-HC1 buffer, and eluted with the same buffer. The fractionation was carried out using an absorbance at 280 nm as an index. The resulting elution pattern is shown in Figure 2. The obtained fractions were divided into three fractions. The biological activities of each fraction were measured, and as a result, the activities were detected in the fraction B-1 (Figure 3 and Table 3).

Table 3

| No. | Sample | TGF-$\beta_1$, binding activity | Activity for promoting NK cell recovery |
|---|---|---|---|
| 1 | a | At 7 mg/mL; Active | At 15 mg/kg; Active |
| 2 | b | At 100 mg/mL; Not active | At 300 mg/kg; Not active |
| 3 | c | At 100 mg/mL; Not active | At 300 mg/kg; Not active |

a: Fraction B-1 obtained by the S-100 gel filtration

b: Fraction B-2 obtained by the S-100 gel filtration

c: Fraction B-3 obtained by the S-100 gel filtration

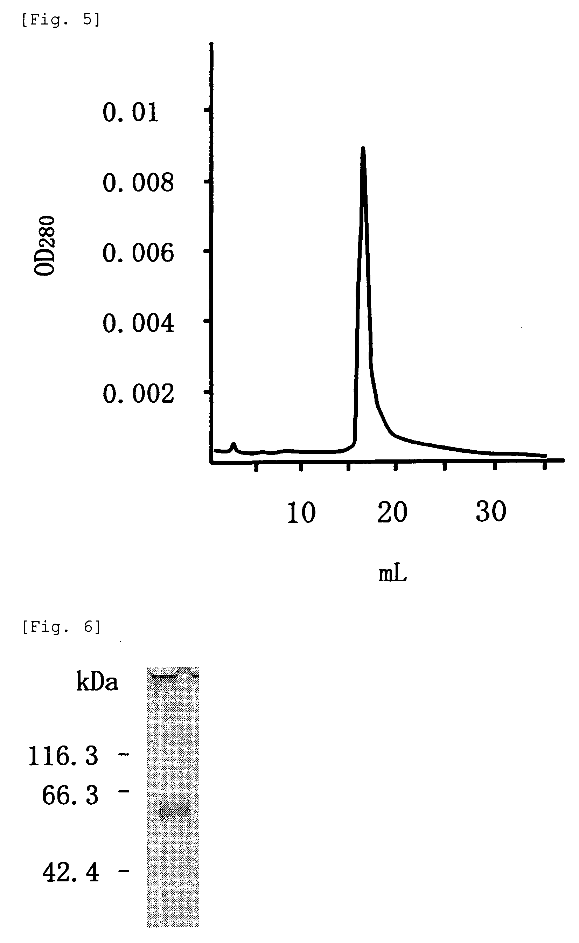

The fraction B-1 was applied to a Sephacryl S-500 (Pharmacia) packed column ($\varphi$ 16 mm, h 600 mm) which had been equilibrated with 0.05 mol/L Tris-HCl buffer, and eluted with the same buffer. The fractionation was carried out using an absorbance at 280 nm as an index. The resulting elution pattern is shown in Figure 4. The obtained fractions were divided into three fractions. The biological activities of each fraction were measured, and as a result, the activities were detected in the fraction B-1-2 (Table 4) .

Table 4

| No. | Sample | TGF-$\beta_1$ binding activity | Activity for promoting NK cell recovery |
|---|---|---|---|
| 1 | a | Not active | At 300 mg/kg; Not active |
| 2 | b | At 3 mg/mL; Active | At 5 mg/kg; Active |

(continued)

| No. | Sample | TGF-$\beta_1$ binding activity | Activity for promoting NK cell recovery |
|-----|--------|-------------------------------|------------------------------------------|
| 3 | c | Not active | At 300 mg/kg; Not active |

a: Fraction B-1-1 obtained by the S-500 gel filtration
b: Fraction B-1-2 obtained by the S-500 gel filtration
c: Fraction B-1-3 obtained by the S-500 gel filtration

The fraction B-1-2 was applied to a C18 reverse phase column, Intesil EP300 (GL Science) packed column ($\varphi$ 4.5 mm, h 150 mm) which had been equilibrated with 0.05 mol/L Tris-HCl buffer, and eluted by a linear gradient using a solution A (0.05% formic acid) and a solution B [acetonitrile/water (90/10)+0.05% formic acid] An absorbance at 280 nm was recorded to obtain a single peak (hereinafter sometimes referred to as PG) as shown in Figure 5. The biological activities were detected in the PG (Table 5). The specific activities in the TGF-$\beta_1$ binding activity and the activity for promoting NK cell recovery were increased by a factor of approximately 33 and a factor of approximately 60, respectively, in comparison with those of the starting substance.

Table 5

| No. | Sample | TGF-$\beta_1$ binding activity | Activity for promoting NK cell recovery |
|-----|--------|-------------------------------|------------------------------------------|
| 1 | a | At 3 mg/mL; Active | At 5 mg/kg; Active |

a: Fraction-1 obtained by the C18 reverse phase chromatography

Example 3: Analyses of physicochemical nature of active substance

(1) Analysis by sodium dodecyl sulfate-polyacrylamide gel electrophoresis (SDS-PAGE)

After 5 $\mu$g of the PG obtained in Example 2 was dissolved in 10 $\mu$L of water, 10 $\mu$L of a buffer for electrophoresis (0.25 mol/L Tris-HCl, 2% SDS, 10% mercaptoethanol, 30% glycerol, and 0.01% bromophenol blue) was further added. The whole was denatured by heating at 65°C for 15 minutes, and applied to electrophoresis. The SDS-PAGE was carried out under the following conditions: Gel: PAG mini "Daiichi" 15/25 (Daiich Pure Chemicals) Buffer for electrophoresis: Tris-glycine running buffer (Daiich Pure Chemicals)
Conditions for electrophoresis: The electrophoresis was performed at 35 mA for 60 minutes.
MW markers: Bands (MW = 97400, 66300, and 42400) (Daiich Pure Chemicals)
Staining: Silver staining
As a result, a spot was detected around the molecular weight of approximately 60 kDa, as shown in Figure 6.
(2) Analysis of amino acid composition
Acidic hydrolysis was carried out as follows. After 6.30 mg of the PG was dissolved in 3.15 mL of pure water, the whole was filtered through a filter (0.22 $\mu$m) to obtain a PG solution (2 mg/mL). To a glass tube, 200 $\mu$L of the PG solution was added, and dried under a reduced pressure by an evaporator to obtain a residue. The residue was dissolved in 200 $\mu$L of 6 mol/L hydrochloric acid, and hydrolyzed at 110°C for 22 hours. The reaction mixture was dried under a reduced pressure by an evaporator to obtain a residue. The residue was dissolved in 100 $\mu$L of purified water, and 50 $\mu$L of the solution was used for an amino acid analysis
Alkaline hydrolysis for analyzing tryptophan was carried out as follows. After 1 mL of the PG solution (2 mg/mL) was added to a plastic tube (Eppendorf tube), the PG solution was dried under a reduced pressure by an evaporator. To the plastic tube, 100 $\mu$L of 4.2 mol/L sodium hydrate solution containing 5 mg of starch soluble were further added. After the plastic tube was placed into a glass tube, hydrolysis was carried out in the sealed and vacuum tube at 110°C for 16 hours. The glass tube was cooled in air and opened. After the plastic tube was cooled on ice, 30 $\mu$L of 6 mol/L hydrochloric acid was added thereto to neutralize the mixture. Into the tube, 420 $\mu$L of purified water was added to adjust the total volume to 500 $\mu$L, and 50 $\mu$L thereof was used for an amino acid analysis.
The quantitative determination was performed by a ninhydrin colorimetry using an amino acid analyzer L-8500 (Hitachi) as equipment.
The amino acid composition was as follows:

Aspartic acid and asparagine 9.65 mol%, threonine 6.15 mol%, serine 6.83 mol%, glutamic acid and glutamine 10.19 mol%, glycine 8.74 mol%, alanine 9.84 mol%, valine 6.94 mol%, 1/2-cystine 0.15 mol%, methionine 1.31 mol%, isoleucine 5.47 mol%, leucine 9.55 mol%, tyrosine 2.64 mol%, phenylalanine 4.09 mol%, lysine 4.99 mol%, histidine 1.97 mol%, arginine 5.00 mol%, tryptophan 1.17 mol%, and proline 5.33 mol%.

(3) Analysis of N-terminus of protein portion
The PG was blotted on a PVDF (polyvinylidene difluoride) membrane by placing a gel after electrophoresis on the PVDF membrane and transferring the PG at 50 mA for 2 hours. As a buffer for blotting, a buffer for electrophoresis supplemented with 10% (v/v) methanol was used. After blotting, the membrane was shaken in a Coomassie brilliant blue (CBB) stain solution [2.5% (w/v) CBB R-250, 25% (w/v) ethanol, and 10% (w/v) acetic acid] for 15 minutes, and decolorized in a decoloring solution [25% (w/v) ethanol and 10% (w/v) acetic acid] until excess dye was removed. The band to be assayed was cut from the membrane and used for an amino acid sequence analysis.
The N-terminal amino acid was analyzed using a gas phase sequencer under the following conditions. The sample was washed with 50% methanol/0.1% trifluoroacetic acid (TFA) and methanol, and dried. The following instruments were used to carry out an Edman degradation and attempt to detect the amino acid sequence from the N-terminus to the 8th amino acid residue.

- Protein sequencer: Procise cLC 492cLC (Applied Biosystem)
- PTH analyzer: 140D (Applied Biosystem)
- UV detector: S200 (Applied Biosystem)
- Program for analysis: Pulsed-liquid ProSorb cLC

As a result, plural phenylthiohydantoin (PTH)-labeled amino acids were detected in each cycle of the Edman degradation, and no increased or decreased PTH-labeled amino acid was detected between cycles. Therefore, the N-terminal sequence of the PG could not be determined.
(4) Circular dichroism analysis (CD)
Purified water was added to approximately 3 mg of the PG, so that a concentration became 2 mg/mL.
As a detector, JASCOJ-500A was used. As a solvent, water was used. The wavelength area was 200 to 250 nm. The cell length was 1 mm. The temperature was room temperature (23°C). The accumulation number was 8. The measurement was performed under the above conditions.
The resulting CD spectrum is shown in Figure 7. As the result of a secondary structure analysis, it was presumed that α-helix was 21%, that β-sheet was 34%, and that the remaining portion was an unordered structure.

.

(5) Analysis of neutral sugars and amino sugars
After 6.30 mg of the PG was dissolved in 3.15 mL of pure water, the whole was filtered through a filter (0.22 μm) to obtain a PG solution (2 mg/mL). To a glass tube, 500 μL of the PG solution was added, and dried under a reduced pressure by an evaporator to obtain a residue. The residue was dissolved in 200 μL of 2 mol/L trifluoroacetic acid, and hydrolyzed at 100°C for 6 hours. The reaction mixture was dried under a reduced pressure by an evaporator to obtain a residue. The residue was dissolved in 200 μL of pure water, and diluted with pure water to 20-fold or 200-fold. After 500 ng of heptose as an internal standard was added to 50 μL of each solution, each mixture was applied to a high performance liquid chromatograph LC-9A (Shimadzu) equipped with a column TSK-gel Sugar AXG (15 cm × 4.6 mm ID; Tosoh) and a spectrophotometer RF-535 (Shimadzu) as a detector. The column temperature was 70°C. The mobile phase was a 0.5 mol/L potassium borate buffer (pH 8.7), and the flow rate thereof was 0.4 mL/min. For the conditions of post-column labeling, 1% arginine/3% boric acid was used as a reaction reagent, the flow rate was 0.5 mL/min., the reaction temperature was 150°C, and the wavelengths for detection were EX 320 nm and EM 430 nm.
The neutral sugar composition in the PG was as follows: Glucose 561.45 μg/mg, mannose 16.68 μg/mg, galactose 42.65 μg/mg, and fucose 9.57 μg/mg, in the order of descending content. Xylose was less than a detectable limit.

To a glass tube, 500 μL of the PG solution was added, and dried under a reduced pressure by an evaporator to obtain a residue. The residue was dissolved in 200 μL of 4 mol/L HC1, and hydrolyzed at 100°C for 6 hours. The reaction mixture was dried under a reduced pressure by an evaporator to obtain a residue. The residue was dissolved in 200 μL of pure water, and a portion (50 μL) thereof was applied to a high performance liquid chromatograph LC-9A (Shimadzu) equipped with a column TSK-gel Sugar SGX (15 cm × 4.6 mm ID; Tosoh) and a spectrophotometer RF-10AXL (Shimadzu) as a detector. The column temperature was 60°C. The mobile phase was a 0.04 mol/L potassium borate buffer (pH 7.6), and the flow rate thereof was 0.3 mL/min. For the conditions of post-column labeling, 1% arginine/3% boric acid was used as a reaction reagent, the flow rate was 0.5 mL/min., the reaction temperature was 150°C, and the wavelengths for detection were EX 320 nm and EM 430 nm.
The amino sugar composition in the PG was as follows: Glucosamine 0.36 $\mu$g/mg. Mannosamine was less than a detectable limit (0.2 $\mu$g/mg).

(6) Determination of proteins
The content of proteins, calculated on the basis of the amounts of amino acids resulting from the PG hydrolysis described in Example 3(2), was 38.42 ug/mg.

(7) Determination of carbohydrates
The content of carbohydrates, calculated on the basis of the amounts of monosaccharides resulting from the PG hydrolysis described in Example 3(5), was 628.72 $\mu$g/mg.

(8) Nuclear magnetic resonance (NMR) analysis

(i) $^1$H one-dimensional NMR analysis of PG
After 2.5 mL of $D_2O$ was added to 11.6 mg of the PG, ultrasonication was carried out for approximately 5 minutes. The sample solution was slightly turbid. The conditions of the $^1$H NMR measurement were as follows. As a detector, Unity plus 500 (Varian) was used. An observation frequency was 500.2 MHz ($^1$H nucleus). As a solvent, $D_2O$ solution (concentration: saturated solution) was used. Acetone 2.225 ppm was used as a standard. The temperatures were 25°C and 45°C. The observation width was 8 KHz. The data point was 64 K. The pulse width was approximately 45°. The pulse repetition time was 5.5 seconds. The accumulation number was 128.

The resulting spectrum is shown in Figure 8. Glucose as a major component among the PG neutral sugars was analyzed. In the $^1$H NMR at 25°C, with respect to 4.5 to 5.5 ppm characteristic of a proton at the 1-position of glucose, it was considered that signals at 5.4 ppm and around 5.2 ppm were derived from a proton at the $\alpha1$ position of glucose. Further, it was considered that minor peaks (doublet peaks) around 4.65 ppm were derived from a proton at the $\beta1$ position. It was presumed that peaks around 4.75 to 5.15 ppm (including a peak at 4.79 ppm observed in the measurement at 45°C) were derived from $\alpha$ protons of carbohydrates other than glucose or proteins.
(ii) $^1$H one-dimensional NMR analysis of PG treated with hydrazine
To analyze the structure of the carbohydrate portion of the PG, the protein portion was removed from the PG with a hydrazine treatment to prepare a sample (hereinafter referred to as hydrazine-treated PG) as follows. The PG was put into a test tube ($\varphi$ 5 mm × h 50 mm), and dried in a vacuum at 50°C for 5 hours using a Hydraclub[R] (SEIKAGAKU Corp., Tokyo). After 2 mL of hydrazine anhydride was added thereto, the whole was heated at 100°C for 2 hours to perform hydrazinolysis in the vapor phase. Hydrazine was evaporated under a reduced pressure, and the residue was dissolved in 0.5 mL of pure water. The solution was dialyzed in pure water overnight. The inner part of the dialyzate was dried under a reduced pressure by an evaporator, to prepare a sample for the NMR measurement. The NMR measurement was carried out under the same conditions as those described in Example 3(8)(i), except that the concentration of the sample was 7.7 mg/2.5 mL.

The resulting spectrum is shown in Figure 9. The $^1$H NMR spectrum at 25°C was broader than that observed in Example 3(8)(i). It was considered that this result was caused by the removal of proteins with the hydrazine treatment and the increased concentration of polysaccharides. Further, the high peak at 3.66 ppm observed in Example 3(8)(i) was not detected.
With respect to 4.5 to 5.5 ppm characteristic of a proton at the 1-position of glucose, it was considered that a signal around 5.4 ppm was derived from a proton at the $\alpha1$ position of glucose. Further, it was presumed that peaks around 4.7 to 5.2 ppm (including a peak at 4.78 ppm observed in the measurement at 45°C) were derived from a proton at the $\beta1$ position or carbohydrates other than glucose.

(iii) $^{13}$C one-dimensional NMR analysis of PG

The PG was dissolved in $D_2O$, so that a concentration became approximately 11.6 mg/2.5 mL. The sample solution was slightly turbid. The conditions of the $^{13}$C NMR measurement were as follows. An observation frequency was 125.8 MHz. Acetone (30.5 ppm) was used as a standard. The temperature was 25°C. The observation width was 31.4 KHz. The data point was 128 K. The pulse width was approximately 45°. The pulse repetition time was 4.0 seconds. The accumulation number was 40000. Measurement was performed under conditions of $^1$H complete decoupling.

The resulting spectrum is shown in Figure 10. In the $^{13}$C NMR, analysis was difficult, because the sensitivity was not increased, and thus, the S/N ratio was insufficient. It was presumed that the high peak at 60.7 ppm was derived from a component other than carbohydrates. Further, it was presumed that carbohydrates contained in the PG were nonuniform, because peaks between 65 to 80 ppm were not uniform.

It was considered that the peak at 100.1 ppm was derived from a carbon at the 1-position of glucose and that the carbon formed an α-type glucoside bond. This result supported the above-mentioned result of the $^1$H NMR.

With respect to positions other than the 1-position, chemical shifts of peaks between 60 to 80 ppm were analyzed. From the analysis of chemical shifts in the glucose monosaccharide or the disaccharide thereof, it was considered that the carbons at the α2 position and the 4-position of glucose formed a glucoside bond. It was presumed that the binding type was an α1-2 bond or an α1-4 bond.

(iv) $^{13}$C one-dimensional NMR analysis of PG treated with hydrazine

The resulting spectrum is shown in Figure 11. In the $^{13}$C NMR, the spectrum had an improved S/N ratio in comparison with the spectrum described in Example 3(8)(iii), and chemical shift values were similar. The high peak at 60.7 ppm detected in Example 3(8)(iii) was lost.

It was presumed that the binding types of carbohydrates were nonuniform, because peaks between 65 to 80 ppm were not uniform. It was considered that the peak at 100.0 ppm was derived from a carbon at the 1-position of glucose and that the carbon formed an α-type glucoside bond. This result supported the above-mentioned result of the $^1$H NMR.

With respect to positions other than the 1-position, chemical shifts of peaks between 60 to 80 ppm were analyzed. From the analysis of chemical shifts in the glucose monosaccharide or the disaccharide thereof, it was considered that the carbons at the α2 position and the 4-position of glucose formed a glucoside bond. It was presumed that the binding type was an α1-2 bond or an α1-4 bond.

(9) Analysis of carbohydrate binding position by methylation

In this example, the carbohydrate portion of the PG was completely methylated with methyl iodide in the presence of powdery sodium hydroxide; the resulting methylated polysaccharide was decomposed into monosaccharides; the resulting methylated monosaccharides were subjected to reductive acetylation for conversion to the acetyl derivative of partially methylated sugar alcohol (partial methylated alditol acetate) were applied to gas chromatography (GC) and gas chromatography/mass spectrometry (GC/MS) measurements to identify the derivatives; and the composition ratio was determined.

More particularly, 2.4 mg of the PG was put into a vial, and dried in the presence of phosphorus pentoxide overnight. After 0.8 mL of dimethyl sulfoxide (DMSO) was added to the vial, the mixture was stirred for 5 hours. Further, 80 mg of powdery sodium hydroxide was added, and the mixture was stirred for 4 hours. To the vial, 0.6 mL of methyl iodide was further added, and the mixture was stirred for 1 hour. The reaction mixture was extracted with 1 mL of chloroform three times, and washed with pure water three times. The chloroform layer was collected, dehydrated by adding thereto 1.8 g of anhydrous sodium sulfate, and allowed to stand at room temperature for 1 hour. The solution was filtered, and the resulting filtrate was concentrated. An aliquot of the concentrated filtrate was spotted on a plate for thin-layer chromatography (TLC). The TLC was carried out, and the presence of methylated saccharide was confirmed by an orcinol-sulfuric acid staining. The remaining concentrated filtrate was dried by an evaporator, and the resulting residue was washed with a small amount of pure water three times. After the residue was dissolved in 0.5 mL of pure water, 0.5 mL of an $NaBH_4$ solution ($NaBH_4$ 20 mg/pure water 2mL) was added thereto. The mixture was stirred, and allowed to stand at room temperature for 4 hours. Excess $NaBH_4$ was decomposed with acetic acid, and the reaction mixture was dried under a reduced pressure by an evaporator. The resulting residue was washed with 2 mL of methanol five times, and 1 mL of a acetic anhydride/pyridine mixture (1/1) was added thereto. The whole was heated at 100°C for 4 hours, and dried under a reduced pressure by an evaporator. The resulting residue was extracted with 2 mL of chloroform, and 2 mL of pure water was added and mixed. The chloroform layer was dehydrated by adding thereto 1.5 g of anhydrous sodium sulfate, allowed to stand at room temperature for 30 minutes, and filtered to obtain a filtrate. The filtrate was dried under a reduced pressure by an evaporator, and the residue was dissolved into chloroform. The solution was used for the following GC and MS analysis.

The conditions of the GC-MS measurement were as follows:

(GC)

GC analyzer: Hewlett-Packard HP5890 SERIES

Column liquid phase: SPB-5 (SUPELCO Japan)

Column: fused silica capillary 30 m $\times$ 0.25 mm ID

Carrier gas: He

Column temperature: 60°C (held for 1 minute) $\rightarrow$ 280°C

(increased at +8°C/minute)

Injection temperature: 280°C

Detection mode: FID

Injection volume: 0.5 $\mu$L

Injection mode: splitless

(MS)

Equipment: JMS DX-303 mass spectrometer (JEOL)

Data analysis system: JMA DA5000 data analysis system (JEOL) Ionization: EI

Electron accelerating voltage: 70 V

ionizing current: 300 $\mu$A

Ion source temperature: 250°C

Ion accelerating voltage: 3.0 KV

Scanning rate: 1 sec/scan (m/z = 10 to 500)

Scanning interval: 1 sec

Each peak derived from the sample on the chromatogram was identified by comparing the peak to standard mass spectra of partially-methylated alditol acetate. The result is shown in Table 6. Further, the composition ratio of each partially-methylated alditol acetate was determined from the peak area calculated from the gas chromatogram. The result is shown in Table 6. These results suggested that the major binding of carbohydrate chains in the PG was 1$\rightarrow$4 linked glucose.

Table 6

|   | A | B | C | D | E | F |
|---|---|---|---|---|---|---|
| 1 | 20.31 | a | $\rightarrow$4 Glc 1$\rightarrow$ | 28543 | 6.63 | 86.33 |
| 2 | 20.33 | b | $\rightarrow$2 Glc 1$\rightarrow$ | 270 | 0.06 | 0.82 |
| 3 | 20.40 | c | $\rightarrow$6 Glc 1$\rightarrow$ | 285 | 0.07 | 0.86 |
| 4 | 21.50 | d | $\rightarrow$4, 6 Glc 1$\rightarrow$ | 2958 | 0.19 | 8.95 |
| 5 | 22.12 | e | $\rightarrow$2,4 Glc 1$\rightarrow$ | 1008 | 0.64 | 3.05 |

A: Retention time (min.)

B: Methylated saccharide

C: Binding position

D: Peak area

E: Composition ratio

F: Composition

a: 2,3,4-tri-O-methylglucose

b: 3,4,6-tri-O-methylglucose

c: 3,4,6-tri-O-methylglucose

d: 2,3-di-O-methylglucose

e: 3,6-tri-0-methylglucose

Glc; Glucose

$\rightarrow$4 Glc 1$\rightarrow$; 1$\rightarrow$4 linked glucose

(continued)

→2 Glc 1→; 1→2 linked glucose
→6 Glc 1→; 1→6 linked glucose
→4, 6 Glc 1→; 1→4 linked and →6 linked glucose
→2,4 Glc 1→; 1→2 linked and →4 linked glucose

(10) Analysis of fatty acids in PG
After 6.65 mg of the PG was put into a 1.5-mL plastic tube, 0.2 mL of methanol was added thereto, and dried under a reduced pressure by a Speed Vac. To a screw-capped glass tube, 6.22 mg of the dried sample was transferred, and 1 mL of 5% HC1-methanol solution was added. The mixture was heated in an oven at 90°C for 16 hours, to convert triglyceride fatty acids contained in the PG to fatty-acid methyl esters. After the methanolysis was completed, 1 mL of hexane was added thereto, and shaken vigorously. After the whole was allowed to stand for a while, the hexane layer was collected, dried by a nitrogen gas purge, and redissolved in 1.0 mL of chloroform (containing approximately 0.01% methyl pentadecanoate as an internal standard). The solution was measured using a GC method as follows.

As standards for a quantitative analysis, 25.37 mg of methyl oleate (GL Science) was added to a 10-mL measuring flask, and chloroform containing the internal standard was added up to the marked line of the measuring flask to prepare a 2537 $\mu$g/mL standard solution. This solution was diluted to 25-fold with chloroform containing the internal standard to prepare a 101.5 $\mu$g/mL solution. Further, this solution was used to prepare a 5-fold-diluted series, i.e., a 20.3 $\mu$g/mL solution, a 4.06 $\mu$g/mL solution, a 0.81 $\mu$g/mL solution. As described below, the GC analysis of these standard solutions was carried out, and a calibration curve was prepared from the relationships between the obtained peak areas and the concentrations thereof. The calibration curve was used to perform a quantitative analysis of each fatty acid contained in the sample solutions.
In addition, as standards for a qualitative analysis, two types of standard solutions (solvent: chloroform) were used as follows:

(1) a mixing solution containing methyl esters of monodivalent to tridivalent unsaturated fatty acids and C14 to C18 saturated fatty acids; and
(2) a mixing solution containing methyl esters of monodivalent unsaturated fatty acids and C20 to C26 saturated fatty acids.

The GC analysis was carried out under the following conditions:

Equipment: Gas chromatograph HP5890 (Hewlett Packard)
Detector: Flame ionization detector (FID)
Column: SP2380 (h 30 m × φ 0.25 mm, thickness of stationary phase film = 0.2 $\mu$m)
Column temperature: 50°C (held for 1 minute) → 250°C (increased at +8°C/minute)
Injection temperature: 250°C
Injection volume: 1 $\mu$L (splitless injection)
In this connection, the GC analysis was carried out as the forms of methyl esters of fatty acids, and therefore, the amounts of fatty acids (as free fatty acids) contained in each sample were calculated in accordance with the following equation:

```
[Amount of fatty acid] = [Measured value as methyl ester of
fatty acid] × [molecular weight of free fatty acid/molecular
weight of methyl ester of fatty acid]
```

As a result, the composition of fatty acids in the PG was as follows: Stearic acid (C18:0) 1.86 $\mu$g/mg, palmitic acid (C16:0) 1.84 $\mu$g/mg, oleic acid [C18:1(9)] 1.52 $\mu$g/mg, linoleic acid [C18:2(9,12)] 0.94 $\mu$g/mg, and myristic acid (C14:0) 0.55 $\mu$g/mg, in the order of descending content.
(9) Infrared spectroscopic analysis (IR)

Infrared spectroscopic analysis was carried out by a KBr method. More particularly, 1 mg of the PG was uniformly mixed with 10 mg of KBr powder, and disks were formed by pressing, and measured using FTIR VAKOR-III (JASCO Corp.). The resulting IR spectrum is shown in Figure 12.

(10) Ultraviolet spectroscopic analysis (UV)

The PG was dissolved in pure water (concentration = 50 mg/mL) and a UV was measured. As a detector, Bio Spec 1600 (Shimadzu) was used. The resulting UV spectrum is shown in Figure 13.

Example 4: Evaluation for antitumor activity
(1) Melanoma B16

Melanoma B16 cells (1x10$^6$ cells/0.2 mL of Hanks balanced salt solution/mouse) were transplanted subcutaneously at an axilla of 6-week-old male C57BL/6N mice [purchased from Charles River Japan (Kanagawa); 6 mice per group). In this connection, melanoma B16 is a tumor cell line (An NY Acad. Sci 100: 762-790, 1963) derived from a tumor developed on the skin of a C57BL/6 mouse. In this example, melanoma B16 cells obtained from Cell Resource Center for Biomedical Research, Institute of Development, Aging and Cancer, Tohoku University (Accession No. TKG0144) and maintained in the axilla of a C57BL/6N mouse in Biomedical Research Laboratories, Kureha Chemical Industry Co. Ltd. were used.

From 24 hours after the subcutaneous transplanting, a predetermined amount (125 mg/kg or 250 mg/kg) of the PG (dissolved in the physiological saline) obtained in Example 2 was intraperitoneally administered every other day, for 10 times in total. As a control group, 0.2 mL of the physiological saline was used instead of the PG solution.

After the transplanting, the survival of the mice was observed every day. The result is shown in Figure 14. The averages of the survival time [mean ± standard error (SE)] in the control group, the PG (125 mg/kg)-administered group, and the PG (250 mg/kg)-administered group were 15.5±1.2 days, 19.0±2.6 days, and 20.5±1.9 days, respectively. The survival time was extended by the PG administration.

(2) Plasmacytoma X5563

Plasmacytoma X5563 cells (1x10$^6$ cells/0.2 mL of Hanks balanced salt solution/mouse) were transplanted subcutaneously at an axilla of 6-week-old female C3H/HeN mice [purchased from Charles River Japan (Kanagawa); 6 mice per group). In this connection, plasmacytoma X5563 is a tumor cell line (JNCI 24: 1153-1165, 1960) derived from a tumor developed in the ileocecum of a C3H/He mouse. In this example, melanoma B16 cells obtained from Cell Resource Center for Biomedical Research, Institute of Development, Aging and Cancer, Tohoku University (Accession No. TKG0174) and maintained in the peritoneal cavity of a C3H/HeN mouse in Biomedical Research Laboratories, Kureha Chemical Industry Co. Ltd. were used.

From 24 hours after the subcutaneous transplanting, a predetermined amount (125 mg/kg or 250 mg/kg) of the PG (dissolved in the physiological saline) obtained in Example 2 was intraperitoneally administered every other day, for 10 times in total. As a control group, 0.2 mL of the physiological saline was used instead of the PG solution.

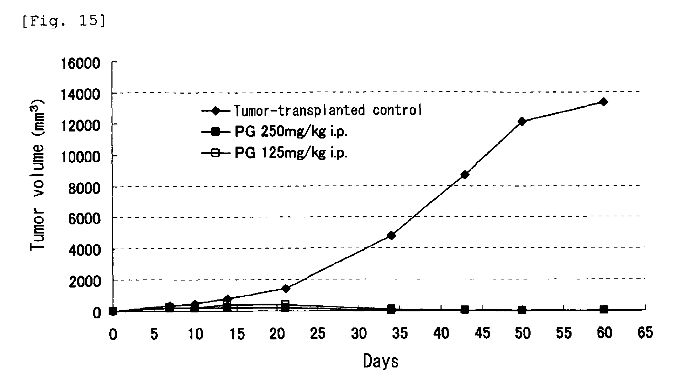

After the transplanting, the survival of the mice was observed every day for 60 days. The survival rates after 60 days in the control group, the PG (125 mg/kg)-administered group, and the PG (250 mg/kg)-administered group were 50% (3/6), 100% (6/6), and 67% (4/6), respectively. The survival rate was extended by the PG administration. Further, as shown in Figure 15, the tumor volume was significantly inhibited by the PG administration.

(3) Sarcoma 180

Sarcoma 180 (S180) cells (1x10$^6$ cells/0.2 mL of Hanks balanced salt solution/mouse) were transplanted subcutaneously at an axilla of 5-week-old female ICR mice [purchased from CLEA Japan, Inc. (Tokyo); 10 mice per group). In this connection, sarcoma 180 is a tumor cell line (Cancer Res 20: 930-939, 1969) derived from a sarcoma derived from an albino mouse. In this example, melanoma B16 cells obtained from Cell Resource Center for Biomedical Research, Institute of Development, Aging and Cancer, Tohoku . University (Accession No. TKG0173) and maintained in the peritoneal cavity of an ICR mouse in Biomedical Research Laboratories, Kureha Chemical Industry Co. Ltd. were used.

From 24 hours after the subcutaneous transplanting, a predetermined amount (125 mg/kg or 250 mg/kg) of the PG (dissolved in the physiological saline) obtained in Example 2 was intraperitoneally administered every other day, for 10 times in total. As a control group, 0.2 mL of the physiological saline was used instead of the PG solution.

On the 25th day after the transplantation, the mice were sacrificed, tumor nodes were taken, and the weights were measured. The weights of tumor nodes (mean $\pm$ SE) in the control group, the PG (125 mg/kg)-administered group, and the PG (250 mg/kg)-administered group were $1.05\pm0.22$ g, $0.01\pm0.01$ g, and $0.00\pm0.00$ g, respectively. The tumor proliferation was significantly inhibited by the PG administration.

(4) Ehrlich carcinoma

Ehrlich carcinoma cells ($1\times10^6$ cells/0.2 mL of Hanks balanced salt solution/mouse) were transplanted subcutaneously at an axilla of 5-week-old female ICR mice [purchased from CLEA Japan, Inc. (Tokyo); 10 mice per group]. In this connection, the Ehrlich carcinoma is a tumor cell line (JNCI 13: 1299-1377, 1953) derived from a tumor developed in mammary glands of a ddY mouse. In this example, Ehrlich carcinoma cells obtained from Cell Resource Center for Biomedical Research, Institute of Development, Aging and Cancer, Tohoku University (Accession No. TKG0147) and maintained in the peritoneal cavity of an ICR mouse in Biomedical Research Laboratories, Kureha Chemical Industry Co. Ltd. were used.

From 24 hours after the subcutaneous transplanting, a predetermined amount (125 mg/kg or 250 mg/kg) of the PG (dissolved in the physiological saline) obtained in Example 2 was intraperitoneally administered every other day, for 10 times in total. As a control group, 0.2 mL of the physiological saline was used instead of the PG solution.

On the 25th day after the transplantation, the mice were sacrificed, tumor nodes were taken, and the weights were measured. The weights of tumor nodes (mean $\pm$ SE) in the control group, the PG (125 mg/kg)-administered group, and the PG (250 mg/kg)-administered group were $1.96\pm0.50$ g, $0.01\pm0.01$ g, and $0.48\pm0.33$ g, respectively. The tumor proliferation was significantly inhibited by the PG administration.

(5) Yoshida sarcoma

Yoshida sarcoma (YS) cells ($1\times10^6$ cells/0.2 mL of Hanks balanced salt solution/mouse) were transplanted intraperitoneally at the peritoneal cavity of 6-week-old male Donryu rats [purchased from Charles River Japan (Kanagawa); 6 mice per group]. In this connection, the Yoshida sarcoma is a tumor cell line (Proc Imp Acad Tokyo 20: 611-618, 1944) derived from a tumor developed by administering o-aminoazotoluene for 3 months and applying arsenic alcohol. In this example, Yoshida sarcoma cells obtained from Cell Resource Center for Biomedical Research, Institute of Development, Aging and Cancer, Tohoku University (Accession No. TKG0088) and maintained in the peritoneal cavity of a Donryu rat in Biomedical Research Laboratories, Kureha Chemical Industry Co. Ltd. were used.

From 24 hours after the intraperitoneal transplanting, a predetermined amount (125 mg/kg or 250 mg/kg) of the PG (dissolved in the physiological saline) obtained in Example 2 was intraperitoneally administered every other day, for 4 times in total. As a control group, 0.2 mL of the physiological saline was used instead of the PG solution.

After the transplanting, the survival of the rats was observed every day. The averages of the survival time (mean $\pm$ SE) in the control group, the PG (125 mg/kg)-administered group, and the PG (250 mg/kg)-administered group were $8.3\pm0.3$ days, $9.0\pm0.4$ days, and $7.5\pm1.1$ days, respectively. The survival time tended to be extended by the PG administration at the dose of 125 mg/kg.

(6) Leukemia P388

Leukemia P388 cells ($1\times10^6$ cells/0.2 mL of Hanks balanced salt solution/mouse) were transplanted subcutaneously at an axilla of 6-week-old CDF1 mice [purchased from Charles River Japan (Kanagawa); 6 mice per group]. In this connection, leukemia P388 is a tumor cell line (Am J Pathol 33: 603, 1957) derived from leukemia caused by treating a DBA/2 mouse with methylcholanthrene. In this example, leukemia P388 cells obtained from Cell Resource Center for Biomedical Research, Institute of Development, Aging and Cancer, Tohoku University (Accession No. TKG0326) and maintained in the peritoneal cavity of a CDF1 mouse in Biomedical Research Laboratories, Kureha Chemical Industry Co. Ltd. were used.

From 24 hours after the subcutaneous transplanting, a predetermined amount (125 mg/kg or 250 mg/kg) of the PG (dissolved in the physiological saline) obtained in Example 2 was intraperitoneally administered every other day, for 4 times in total. As a control group, 0.2 mL of the physiological saline was used instead of the PG solution.

After the transplanting, the survival of the mice was observed every day. The averages of the survival time (mean ± SE) in the control group, the PG (125 mg/kg)-administered group, and the PG (250 mg/kg)-administered group were 17.3±2.2 days, 18.3±2.4 days, and 19.6±1.7 days, respectively. The survival time tended to be extended by the PG administration.

(7) Leukemia EL4

Leukemia EL4 cells (1x10$^6$ cells/0.2 mL of Hanks balanced salt solution/mouse) were transplanted subcutaneously at an axilla of 6-week-old male C57BL/6N mice [purchased from Charles River Japan (Kanagawa); 6 mice per group). In this connection, leukemia EL4 is a tumor cell line (Cancer Res 16: 338-343, 1956) derived from lymphoma developed in the pancreas by treating a mouse with dimethylbenzanthracene (DMBA). In this example, leukemia EL4 cells obtained from Cell Resource Center for Biomedical Research, Institute of Development, Aging and Cancer, Tohoku University (Accession No. TKG0150) and maintained in the peritoneal cavity of a C57BL/6N mouse in Biomedical Research Laboratories, Kureha Chemical Industry Co. Ltd. were used.

From 24 hours after the subcutaneous transplanting, a predetermined amount (125 mg/kg or 250 mg/kg) of the PG (dissolved in the physiological saline) obtained in Example 2 was intraperitoneally administered every other day, for 4 times in total. As a control group, 0.2 mL of the physiological saline was used instead of the PG solution.

After the transplanting, the survival of the mice was observed every day. The averages of the survival time (mean ± SE) in the control group, the PG (125 mg/kg)-administered group, and the PG (250 mg/kg)-administered group were 12.0±0.4 days, 13.0±0.0 days, and 11.8±0.3 days, respectively. The survival time tended to be extended by the PG administration at the dose of 125 mg/kg.

(8) Hepatoma AH13

Hepatoma AH13 cells (1x10$^6$ cells/0.2 mL of Hanks balanced salt solution/mouse) were transplanted intraperitoneally at the peritoneal cavity of 6-week-old male Donryu rats [purchased from Charles River Japan (Kanagawa); 6 mice per group). In this connection, hepatoma AH13 is a tumor cell line (Journal of Japanese Society of Pathology 11: 147-168, 1967) derived from a tumor developed in the liver by treating a rat with dimethylaminoazobenzene (DAB). In this example, hepatoma AH13 cells obtained from Cell Resource Center for Biomedical Research, Institute of Development, Aging and Cancer, Tohoku University (Accession No. TKG0011) and maintained in the peritoneal cavity of a Donryu rat in Biomedical Research Laboratories, Kureha Chemical Industry Co. Ltd. were used.

From 24 hours after the intraperitoneal transplanting, a predetermined amount (125 mg/kg or 250 mg/kg) of the PG (dissolved in the physiological saline) obtained in Example 2 was intraperitoneally administered every other day, for 4 times in total. As a control group, 0.2 mL of the physiological saline was used instead of the PG solution.

After the transplanting, the survival of the rats was observed every day. The survival rates after 12 days in the control group, the PG (125 mg/kg)-administered group, and the PG (250 mg/kg)-administered group were 17% (1/6), 67% (4/6), and 67% (4/6), respectively. The survival rate was extended by the PG administration.

INDUSTRIAL APPLICABILITY

The glycoprotein of the present invention may be applied to the use of an immune enhancing agent, an agent for promoting a recovery from stress, and/or an antitumor agent.

Although the present invention has been described with reference to specific embodiments, various changes and modifications obvious to those skilled in the art are possible without departing from the scope of the appended claims.

BRIEF DESCRIPTION OF THE DRAWINGS

Figure 1 is a chromatogram showing an elution pattern obtained when the aqueous layer resulting from a ChMe treatment of an alkaline solution extract was applied to an anion exchange chromatography.

Figure 2 is a chromatogram showing an elution pattern obtained when the Fraction B resulting from the anion exchange chromatography was applied to a gel filtration chromatography (Sephacryl S-100).

Figure 3 is a graph showing the TGF-$\beta_1$ binding activity in each fraction obtained by the gel filtration chromatography (Sephacryl S-100) of the Fraction B resulting from the anion exchange chromatography.

Figure 4 is a graph showing an elution pattern obtained when the Fraction B-1 resulting from the gel filtration chromatography (Sephacryl S-100) was further applied to another gel filtration chromatography (Sephacryl S-500),

and the TGF-$\beta_1$ binding activity in each fraction resulting therefrom.

Figure 5 is a chromatogram showing an elution pattern obtained when the Fraction B-1-2 resulting from the gel filtration chromatography (Sephacryl S-500) was further applied to a reversed phase chromatography.

Figure 6 shows the result of SDS-polyacrylamide gel electrophoresis of the glycoprotein of the present invention obtained by the reversed phase chromatography.

Figure 7 is a CD spectrum obtained by a circular dichroism analysis of the glycoprotein of the present invention.

Figure 8 is a spectrum obtained by a $^1$H one-dimensional NMR measurement of the glycoprotein of the present invention.

Figure 9 is a spectrum obtained by a $^1$H one-dimensional NMR measurement of the glycoprotein treated with hydrazine.

Figure 10 is a spectrum obtained by a $^{13}$C one-dimensional NMR measurement of the glycoprotein of the present invention.

Figure 11 is a spectrum obtained by a $^{13}$C one-dimensional NMR measurement of the glycoprotein treated with hydrazine.

Figure 12 is a spectrum obtained by an infrared spectroscopic analysis of the glycoprotein of the present invention.

Figure 13 is a spectrum obtained by an ultraviolet spectroscopic analysis of the glycoprotein of the present invention.

Figure 14 is a graph showing the survival rates of melanoma-B16-implanted mice to which the glycoprotein of the present invention was administered.

Figure 15 is a time course showing the tumor volumes of plasmacytoma-X5563-implanted mice to which the glycoprotein of the present invention was administered.

**Claims**

1. A glycoprotein having the following properties:

    (a) molecular weight: approximately 60 kDa (determined by sodium dodecyl sulfate-polyacrylamide gel electrophoresis), and
    (b) ratio of carbohydrate content to protein content: 16.4:1.0 (carbohydrate:protein).

2. A glycoprotein obtainable by a process comprising the steps of:

    (1) extracting *Tricholoma matsutake* with an alkaline solution or hot water,
    (2) adsorbing the resulting extract by an anion exchange resin,
    (3) eluting the adsorption fraction from the anion exchange resin with an eluting solution, and
    (4) fractionating from the eluted fraction obtained by the eluting step a fraction having a molecular weight of 50 to 70 kDa by gel filtration.

3. A pharmaceutical composition comprising the glycoprotein according to claim 1 or 2, and a pharmaceutically or veterinarily acceptable carrier or diluent.

4. An immune enhancing agent, **characterized by** comprising as an active ingredient the glycoprotein according to claim 1 or 2.

5. An agent for promoting a recovery from stress, **characterized by** comprising as an active ingredient the glycoprotein according to claim 1 or 2.

6. An antitumor agent, **characterized by** comprising as an active ingredient the glycoprotein according to claim 1 or 2.

7. A method for an immune enhancement, comprising administering to a subject in need thereof the glycoprotein according to claim 1 or 2 in an amount effective therefor.

8. A method for promoting a recovery from stress, comprising administering to a subject in need thereof the glycoprotein according to claim 1 or 2 in an amount effective therefor.

9. A method for treating or preventing a tumor, comprising administering to a subject in need thereof the glycoprotein according to claim 1 or 2 in an amount effective therefor.

**10.** Use of the glycoprotein according to claim 1 or 2 in the manufacture of an immune enhancing agent or a pharmaceutical composition for an immune enhancement.

**11.** Use of the glycoprotein according to claim 1 or 2 in the manufacture of an agent for promoting a recovery from stress or a pharmaceutical composition for promoting a recovery from stress.

**12.** Use of the glycoprotein according to claim 1 or 2 in the manufacture of an antitumor agent or a pharmaceutical composition for treating or preventing a tumor.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

## INTERNATIONAL SEARCH REPORT

| International application No. |
|---|
| PCT/JP2004/015057 |

A. CLASSIFICATION OF SUBJECT MATTER
    Int.Cl$^7$  C07K14/75, A61K38/00, A61P35/00, A61P37/04, A61P43/00//
             C12P21/00

According to International Patent Classification (IPC) or to both national classification and IPC

B. FIELDS SEARCHED

Minimum documentation searched (classification system followed by classification symbols)
    Int.Cl$^7$  C07K1/00-19/00, A61K31/00-48/00, A61P1/00-43/00, C12P21/00-08

Documentation searched other than minimum documentation to the extent that such documents are included in the fields searched

Electronic data base consulted during the international search (name of data base and, where practicable, search terms used)
    WPI(STN), BIOSIS(STN), MEDLINE(STN), CA(STN)

C. DOCUMENTS CONSIDERED TO BE RELEVANT

| Category* | Citation of document, with indication, where appropriate, of the relevant passages | Relevant to claim No. |
|---|---|---|
| A | WO 03/70264 A1  (KUREHA CHEMICAL INDUSTRY CO., LTD.), 28 August, 2003 (28.08.03), & AU 2003211434 A1 | 1-6,10-12 |
| A | WO 02/30440 A1  (KUREHA CHEMICAL INDUSTRY CO., LTD.), 18 April, 2002 (18.04.02), & EP 1331009 A1          & US 2003/0180901 A1 | 1-6,10-12 |
| P,A | US 2004/0126392 A1  (KUREHA CHEMICAL INDUSTRY CO., LTD.), 01 July, 2004 (01.07.04), & JP 2004-210695 A | 1-6,10-12 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 October, 2004 (28.10.04) | 16 November, 2004 (16.11.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

**INTERNATIONAL SEARCH REPORT**

| International application No. |
| --- |
| PCT/JP2004/015057 |

C (Continuation).   DOCUMENTS CONSIDERED TO BE RELEVANT

| Category* | Citation of document, with indication, where appropriate, of the relevant passages | Relevant to claim No. |
| --- | --- | --- |
| P,A | US 2004/0126393 A1 (KUREHA CHEMICAL INDUSTRY CO., LTD.), 01 July, 2004 (01.07.04), & JP 2004-210694 A | 1-6,10-12 |
| A | WO 01/49308 A1 (KUREHA CHEMICAL INDUSTRY CO., LTD.), 12 July, 2001 (12.07.01), & EP 1256351 A1         & US 2003/044424 A1 | 1-6,10-12 |

Form PCT/ISA/210 (continuation of second sheet) (January 2004)

## INTERNATIONAL SEARCH REPORT

| International application No. |
|---|
| PCT/JP2004/015057 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 7−9
   because they relate to subject matter not required to be searched by this Authority, namely:
   The inventions as set forth in claims 7 to 9 are substantially relevant to methods for treatment of the human body by therapy and thus relate to a subject matter which this International Searching Authority is not required, under the provisions of Article 17(2)(a)(i)   (continued to extra sheet.)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.

PCT/JP2004/015057

Continuation of Box No.II-1 of continuation of first sheet(2)

of the PCT and Rule 39.1(iv) of the Regulations under the PCT, to search.

Form PCT/ISA/210 (extra sheet) (January 2004)